Europäisches Patentamt

European Patent Office

Office européen des brevets

(11) Veröffentlichungsnummer: **0 289 973 B1**

(12) **EUROPÄISCHE PATENTSCHRIFT**

(45) Veröffentlichungstag der Patentschrift: **21.08.91**

(51) Int. Cl.⁵: **C07C 409/24**, C07C 55/18

(21) Anmeldenummer: **88106986.8**

(22) Anmeldetag: **30.04.88**

(54) **Verwendung eines fetten Öles ex Helianthus annuus zur Herstellung von Diperazelainsäure.**

(30) Priorität: 08.05.87 DE 3715464

(43) Veröffentlichungstag der Anmeldung:
09.11.88 Patentblatt 88/45

(45) Bekanntmachung des Hinweises auf die
Patenterteilung:
21.08.91 Patentblatt 91/34

(84) Benannte Vertragsstaaten:
DE FR GB IT

(56) Entgegenhaltungen:
EP-A- 0 127 782
FR-A- 2 461 744
US-A- 4 627 192

KIRK-OTHMER "Encyclopedia of chemical
technology", 3. Ausgabe, Band 16, 1981,
Wiley-Interscience Publication, New York,
US

(73) Patentinhaber: **Henkel Kommanditgesellschaft
auf Aktien**
**Postfach 1100 Henkelstrasse 67**
**W-4000 Düsseldorf-Holthausen(DE)**

(72) Erfinder: **Eierdanz, Horst, Dr.**
**Verbindungsstrasse 5**
**W-4010 Hilden(DE)**
Erfinder: **Schulz, Paul, Dr.**
**Auf dem Scheidt 35**
**W-5600 Wuppertal 1(DE)**
Erfinder: **Kottwitz, Beatrix, Dr.**
**Urdenbacher Allee 51**
**W-4000 Düsseldorf 13(DE)**

## Beschreibung

Die Erfindung betrifft die Verwendung eines fetten Öles ex Helianthus annuus mit einem Ölsäuregehalt von 78 bis 92 Gew. -% und einem Linolsäuregehalt von 2 bis 8 Gew. -%, jeweils bezogen auf enthaltende Gesamtfettsäuren, Rest (auf 100 Gew.-%) gegebenenfalls Palmitinsäure und Stearinsäure sowie bis zu 1 Gew.-% Fettsäuren aus der Myristin-, Palmitolein-, Linolen-, Arachin-, Eikosen- und Erucasäure gebildeten Gruppe unter Ausschluß von Anreicherungsschritten bezüglich des Ölsäuregehaltes zur Herstellung von Diperazelainsäure durch

a) Fettspaltung,
b) Destillation,
c) oxidative Ozonolyse des so erhaltenen Fettsäuregemisches und
d) Umsetzung der so erhaltenen Azelainsäure mit Wasserstoffperoxid zu Diperazelainsäure.

Unter "Destillation" ist eine einfache Überkopfdestillation ohne Fraktionierung des Destillats zu verstehen.

Die Herstellung von Azelainsäure durch oxidative Ozonolyse von technischer Ölsäure ist aus den US-PS 24 50 858 und 28 13 113 bekannt. Weiterhin bekannt ist die Herstellung von Diperazelainsäure durch Umsetzung derselben mittels Wasserstoffperoxid in Gegenwart von Schwefelsäure, vgl. D. Swern et al., J. Am. Chem. Soc., 79, 1929 ff (1957) ; M. Dankowski, EP-A 127 782.

Bei der Herstellung von Azelainsäure aus technischer Ölsäure hat sich gezeigt, daß sich die Anwesenheit von mehrfach ungesättigten Fettsäuren in den eingesetzten technischen Ölsäuren infolge des verstärkten Auftretens von Nebenprodukten ungünstig auswirkt. Darüber hinaus wird die üblicherweise eingesetzte technische Ölsäure durch Anreicherung (Lösemittel- oder Umnetzverfahren) und Aufreinigung der in Talgspaltfettsäure enthaltenen Ölsäure gewonnen. Den Anreicherungsverfahren zur Gewinnung der Ölsäure sind allerdings prinzipielle Grenzen gesetzt. Technisch kommen daher (65 bis 70 %-ige Ölsäuren bei der Herstellung von Azelainsäure zum Einsatz.

Es wurde nun gefunden, daß sich aus einem fetten Öl einer Helianthus annuus-Sorte, wie sie z. B. in der US-PS 4 627 192 beschrieben ist, eine Azelainsäure in hoher Ausbeute und Reinheit erhalten läßt. Überraschenderweise weist die aus der so erhaltenen Azelainsäure hergestellte Diperazelainsäure eine höhere Stabilität als diejenige auf, die gemäß dem Stand der Technik über eine mittels aufwendiger Verfahren angereicherte und aufgereinige Talgspaltfettsäure hergestellt wurde, obwohl die Talgspaltfettsäure mit 7 bis 10 % einen vergleichbar hohen Anteil an zweifach ungesättigten Fettsäuren wie die erfindungsgemäß einzusetzende Fettsäure ex Helianthus annuus aufweist.

Die erfindungsgemäß zu verwendenden fetten Öle ex Helianthus annuus sind Naturprodukte, so daß sie hinsichtlich der Zusammensetzungen ihrer Bestandteile gewissen Schwankungen unterliegen. Üblicherweise für die Erfindung geeignete fette Öle können hinsichtlich ihrer Hauptkomponenten folgende Zusammensetzungen aufweisen:

Ölsäure          78 bis 92 Gew.-%
Linolsäure        2 bis 10 Gew.-%
Palmitinsäure      2 bis 5 Gew.-%
Stearinsäure       2 bis 7 Gew.-%

Darüber hinaus hat sich gezeigt, daß die Reinigung einer unter Verwendung der fetten Öle der Erfindung erhaltenen Diperazelainsäure wesentlich einfacher ist; auch die als Zwischenstufe erhaltene reine Azelainsäure läßt sich durch einfache Kristallisation, z.B. aus unpolaren Lösemitteln oder aus Wasser, reinigen. Darüber hinaus ist bei der Ozonolyse von Ölsäure aus dem erfindungsgemäß einzusetzenden fetten Öl der eingangs genannten Zusammensetzung der Ozonverbrauch deutlich erniedrigt.

Die Erfindung wird im folgenden anhand eines bevorzugten Ausführungsbeispiels näher erläutert.

Beispiel.

Ausgangsmaterial war ein Fettsäuregemisch ex Helianthus annuus mit der folgenden, gaschromatografisch ermittelten Zusammensetzung:

Myristinsäure     0,1 Gew.-%
Palmitinsäure      3,1 Gew.-%
Stearinsäure       2,0 Gew.-%
Ölsäure           86,2 Gew.-%
Linolsäure         7,8 Gew.-%
Linolensäure       0,2 Gew.-%
Arachinsäure       0,2 Gew.-%

Eikosensäure     0,2 Gew.-%
Erucasäure     0,2 Gew.-%

4570 g/h einer 20 %-igen Lösung des obigen Fettsäuregemisches in Pelargonsäure, 915 g/h Wasser, das mit der vorstehenden organischen Phase vor dem Eintritt in die Reaktionskolonne in einem dynamischen Mischer intensiv homogenisiert wurde, und 140 g/h Ozon in Form eines 3 %-igen Ozon-Luft-Gemisches wurden in eine Edelstahlkolonne mit Mellapack-Füllung im Gleichstrom von oben nach unten eingespeist. Die Reaktionstemperatur wurde durch Verdunstung eines Hauptteils der Wassermenge im Trägerluftstrom bei ca. 30°C stabilisiert. Das Reaktionsprodukt ergab nach Phasentrennung eine organische Schicht, die anschließend in einem Glas- oder Emailreaktor in 3 Stufen oxidativ aufgearbeitet wurde:

1. durch Luftoxidation bei 80°C (70 l Luft/h/kg Reaktionsgemisch), wobei die Oxidationsluft vor dem Einleiten in den Reaktor bei 60°C mit Wasserdampf abgesättigt wurde; die Luftoxidation dauerte ca. 1,5 Stunden.

2. Nachoxidation mit Wasserstoffperoxid (28,5 g 70 %-ige $H_2O_2$-Lösung/kg Reaktionsgemisch) bei 90°C; die Nachoxidation dauerte ca. 2 Stunden.

3. thermischer Peroxidabbau bei 130°C (ca. 1 Stunde).

Die ausreagierte Mischung (Peroxid-Zahl < 20) wurde zunächst am Dünnschichtverdampfer (Arbeitsdruck 1 mbar, Wärmeträgertemperatur: 160°C) von der als Lösemittel eingesetzten und durch Ölsäurespaltung entstandenen Pelargonsäure getrennt. Die Rückstandsschmelze (ca. 80 % Azelainsäure) wird im Kristallisationsmedium (Toluol oder Wasser) heiß gelöst (ca. 95°C, Verhältnis 1 : 1) und unter definierter Rührung gekühlt. Das getrocknete Kristallisat (93 bis 95 % Azelainsäure) entsprach einer Ausbeute von 85 bis 87 %, bezogen auf eingesetzte Ölsäure. Es ist unmittelbar, d.h. ohne zusätzliche Reinigung, für die Diperazelainsäure-Herstellung einsetzbar.

Die Herstellung der Diperazelainsäure erfolgte entsprechend den obengenannten Veröffentlichungen, J. Am. Chem. Soc. 79, 1929 ff (1957); M Dankowski, EP 127 762.

Die so gewonnene Diperazelainsäure kann als wirksames Bleichmittel bei niedrigen Waschtemperaturen (30 bis 60°C) eingesetzt werden. Sie zeigte sowohl in Reinsubstanz als auch phlegmatisiert mit Natriumsulfat eine verbesserte Lagerstabilität bei 25°C, wie sich aus den folgenden Tabellen ergibt.

Tabelle 1

Lagerstabilität einer erfindungsgemäß erhaltenen Diperoxy-azelainsäure.

| Tage | Reinsubstanz | 30 %-ig in $Na_2SO_4$ |
|---|---|---|
| | | % Zersetzung |
| 10 | stabil | stabil |
| 27 | 2 % | 2 % |
| 46 | 5 % | 3 % |

Tabelle 2

Lagerstabilität einer Diperoxyazelainsäure, hergestellt aus Talgspaltfettsäure

| Tage | Reinsubstanz | 30 %-ig in $Na_2SO_4$ |
|---|---|---|
| | | % Zersetzung |
| 12 | nur bei + 4°C | 4 % |
| 33 | längere Zeit | 4 - 8 % |
| 50 | lagerbar | 6 - 8 % |

In Tabelle 3 sind die mit Talgspaltfettsäure und erfindungsgemäß erzielbaren Ausbeuten an Azelainsäure sowie deren Reinheitsgrade zusammengefaßt.

## Tabelle 3

Azelainsäureausbeuten (bezogen auf eingesetzte Fettsäure) und Reinheitsgrade

| | Einsatzstoff | |
|---|---|---|
| | Talgspaltfettsäure | Beispiel |
| Ausbeute | 82,4 % | 85,5 % |
| Azelainsäuregehalt im Endprodukt* | ca. 83 % | ca. 95 % |

\* einfache Umkristallisation aus Toluol

In Tabelle 4 wird der Ozonverbrauch beim Einsatz von Talgfettsäure gemäß Stand der Technik und gemäß der Erfindung verglichen.

## Tabelle 4

Ozonverbrauch beim Einsatz unterschiedlicher Oleine.

| | Einsatzstoff | |
|---|---|---|
| | Talgspaltfettsäure** | Beispiel** |
| Ozonverbrauch (kg Ozon pro kg Azelainsäure) | 0,493 | 0,323 |

\*\* Azelainsäure-Ausbeuten s. Tab. 1, Ozonüberschuß jeweils ca. 5 %.

## Patentansprüche

1. Verwendung eines fetten Öles ex Helianthus annuus mit einem Ölsäuregehalt von 78 bis 92 Gew.-% und einem Linolsäuregehalt von 2 bis 8 Gew.-%, jeweils bezogen auf enthaltende Gesamtfettsäuren, Rest (auf 100 Gew.-%) gegebenenfalls Palmitinsäure und Stearinsäure sowie bis zu 1 Gew.-% Fettsäuren aus den von Myristin-, Palmitolein-, Linolen-, Arachin-, Eikosen- und Erucasäure gebildeten Gruppe unter Ausschluß von Anreicherungsschritten bezüglich des Ölsäuregehaltes zur Herstellung von Diperazelainsäure durch
   a) fettspaltung,
   b) Destillation,

c) oxidative Ozonolyse des so erhaltenen Fettsäuregemisches und

d) Umsetzung der so erhaltenen Azelainsäure mit Wasserstoffperoxid zu Diperazelainsäure.

## Claims

1. The use of a fatty oil ex Helianthus annuus containing 78 to 92% by weight oleic acid and 2 to 8% by weight linoleic acid, based on total fatty acids present, balance (to 100% by weight) optionally palmitic acid and stearic acid and up to 1% by weight fatty acids from the group comprising myristic, palmitoleic, linolenic, arachic, eicosenoic and erucic acid, with elimination of enrichment steps in respect of the oleic acid content, for the production of diperazelaic acid by

   a) fat splitting,

   b) distillation,

   c) oxidative ozonolysis of the fatty acid mixture obtained and

   d) reaction of the azelaic acid thus obtained with hydrogen peroxide to form diperazelaic acid.

## Revendications

1. Utilisation d'une huile grasse d'Helianthus annuus ayant une teneur en acide oléique de 78 à 92 % en poids et une teneur en acide linoléique de 2 à 8 % en poids, respectivement rapporté aux acides gras totaux qui y sont contenus, le reste (à 100 % en poids) constitué le cas échéant d'acide palmitique, d'acide stéarique ainsi que jusqu'à 1 % en poids d'acides gras choisis dans le groupe qui consiste en l'acide myristique, l'acide palmitoléique, l'acide linolénique, l'acide arachidique, l'acide eicosaénoïque et l'acide érucique, à l'exclusion des étapes d'enrichissement en ce qui concerne la teneur en acide oléique, en vue de l'obtention d'acide diperazélaïque par :

   a) par coupure des graisses

   b) distillation

   c) ozonolyse oxydante du mélange d'acides gras ainsi obtenu

   d) mise en réaction de l'acide azélaïque ainsi obtenu avec du peroxyde d'hydrogène pour former l'acide diperazélaïque.